# EUROPEAN PATENT APPLICATION

(11) **EP 3 031 345 A1**
(43) Date of publication of application: **15.06.2016**
(21) Application number: 14758411.4
(22) Date of filing: 04.08.2014
(51) Int. Cl.: A45C 11/00, A45C 13/26, A45F 5/00

(54) **CASE FOR MOBILE PHONES OR SIMILAR DEVICES**

(30) Priority: 06.08.2013 ES 201330976 U
(71) Applicant: Erlandsen Strange, Paul Anthony, 08028 Barcelona (ES)
(72) Inventor: Erlandsen Strange, Paul Anthony, 08028 Barcelona (ES)
(74) Representative: Isern-Jara, Nuria
(86) International application number: PCT/ES2014/070636
(87) International publication number: WO 2015/018960

(57) **Abstract**

Case for mobile phones or similar devices (10), which is formed by a body (1) made of any suitable material having a configuration that fits the mobile phone (10) to be housed, comprising a foldaway handle (2) which protrudes from the lower body (1) of the case, and enables the user to use the phone or mobile device (10) in certain situations.

## Description

### OBJECT OF THE INVENTION

The object of the present invention application is the registration of a case for mobile phones which incorporates significant innovations and advantages.

More specifically, the invention proposes the development of a case that enables the user to use mobile phones or devices in certain situations.

### BACKGROUND OF THE INVENTION

Cases or covers for mobile phones are elements that serve to protect the electronic mobile device from possible blows or wear and tear from everyday use. There is currently a wide variety of cases available on the market, which essentially consist of a partial cover that makes it possible to leave the screen of the device and the buttons of the mobile phone uncovered to facilitate user handling.

These cases can be manufactured from different materials (plastic, silicone, a combination thereof, etc.) such that they may have a rigid, semi-rigid or soft configuration, in addition to an infinite variety of designs, shapes and colours.

It is well known that mobile phones known as smartphones include cameras which enable them to take photos or shoot videos and are essentially activated by pressing a corresponding icon. Mention should be made of the fact that the photographing function is usually performed using both hands, as this is the manner in which to hold the mobile telephone with greater manoeuvrability and security. However, when the user is obliged to use it with only one hand, whether to take a self-portrait or due to having the other hand occupied for other reasons, it becomes difficult to hold and operate at the same time, resulting in poor-quality photos or even dropping the mobile phone.

### DESCRIPTION OF THE INVENTION

The present invention has been developed for the purpose of providing a case configured as a novelty within the field of application and that resolves the aforementioned drawbacks, as well as providing other additional advantages that will be evident from the accompanying description below.

Therefore, one object of the present invention is to provide a new case for mobile phones consisting of a body made of any type of suitable material having a configuration that fits the mobile phone to be housed therein, and is characterised in that it comprises a foldaway handle that protrudes from the lower part of the body of the case which allows the user to hold and manipulate the mobile phone or device with only one hand, providing security and balance. Therefore, when the handle is fully folded, said case has the characteristics of any conventional case, such that its size, shape or texture do not change and do not inconvenience the user.

In one embodiment of the invention, the rear face of the body of the case comprises guiding means through which the handle is susceptible of sliding longitudinally.

Preferably, the guiding means comprise a pair of longitudinal guides separated from one another, between which the handle slides, said handle having abutting means for limiting the travel path. The presence of these guides gives the case greater rigidity.

In an alternative embodiment of the invention, one end of the handle comprises a rotary shaft articulated in a hinged manner to the body of the case.

In another preferred embodiment of the invention, the body of the case may include a shaft whereto the handle is coupled in a swivelling manner, such that said handle is capable of rotating on a plane parallel to the plane defined by the body of the case.

In accordance with another aspect of the present invention, the body of the case has a housing having dimensions that complement the dimensions of the handle wherein said handle may be disposed in a non-unfolded condition.

In a preferred embodiment, the housing consists of a through-hole on the rear face of the body of the case.

According to another characteristic, the handle is made of a rigid or semi-rigid material.

Other characteristics and advantages of the case object of the present invention will be evident from the description of a preferred but non-exclusive embodiment, illustrated by way of non-limiting example in the accompanying drawings, wherein:

### BRIEF DESCRIPTION OF THE DRAWINGS

Figures 1 a to 1 c show perspective views of a first embodiment of a case in accordance with the invention;
Figures 2a to 2d show front perspective views of a second embodiment of a case in accordance with the invention;
Figures 3a to 3c show rear perspective views of a third embodiment of a case in accordance with the invention; and
Figure 4 shows a perspective view of the user holding a mobile phone incorporating the case of the invention.

### DESCRIPTION OF A PREFERRED EMBODIMENT

In light of the aforementioned figures and in accordance with the numbering adopted, a preferred embodiment of the invention can be observed therein which comprises the parts and elements indicated and described in detail below.

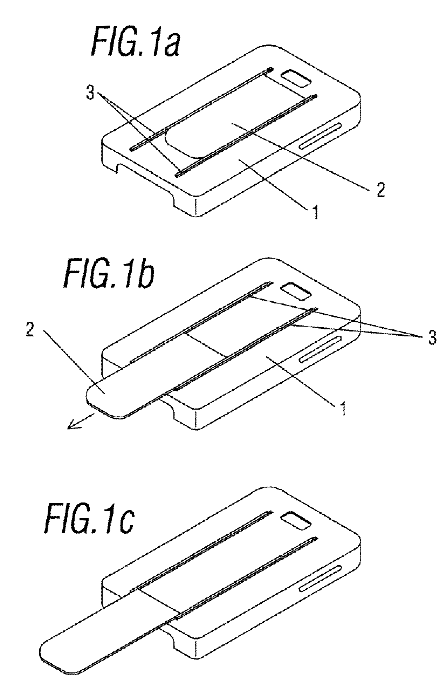

Thus, as can be observed in figures 1 a to 1 c, a case appropriate for being disposed on a mobile telephone device (10) has been represented that essentially comprises a body (1) having a substantially rectangular base which incorporates a foldaway handle (2) that protrudes from the lower part of the body (1) of the case. In particular, a pair of parallel longitudinal guides (3) separated from one another are provided on the rear face of the body (1) of the case, between which the handle (2) slides in the direction indicated by the arrow in figure 1b, adopting a folded or unfolded position. Also, in order to prevent the foldaway handle (2) from becoming separated from the body (1) of the case, the foldaway handle (2) includes means for limiting the travel path.

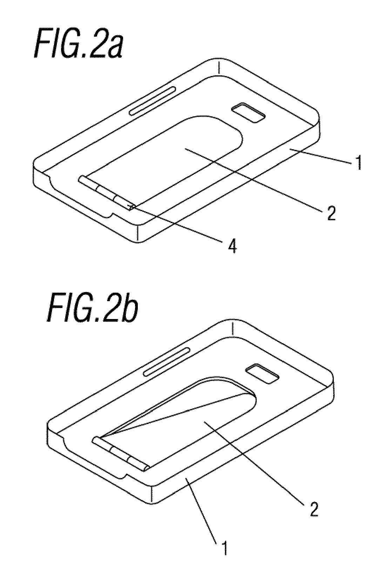

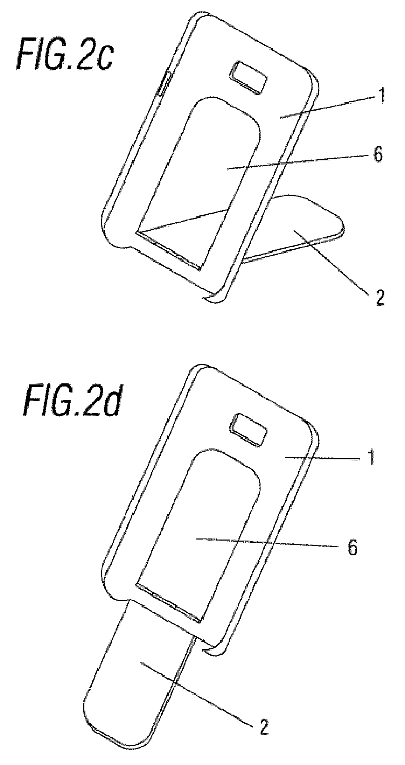

In an alternative embodiment of the figure represented in figures 2a to 2d and wherein the components themselves have the same numerical reference, one end of the handle comprises a rotary or swiveling shaft (4) that is articulated in a hinged manner to the body (1) of the case. Advantageously, the foldaway handle (2) can adopt different positions with respect to the body (1), such that in one of the positions, as opposed to the previously described embodiment, the telephone can be disposed in an inclined manner and on a surface, such as for example a table or desk, so as to facilitate handling thereof.

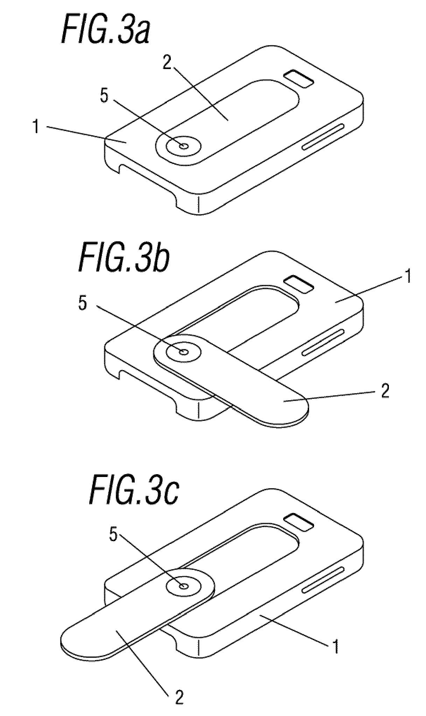

Lastly, figures 3a to 3c show a third embodiment wherein the common components or elements also have the same numerical reference, wherein, as opposed to the previous embodiments, the body (1) of the case includes a shaft or pin (5) whereto the foldaway handle (2) is coupled in a swiveling manner, such that said handle (2) is capable of rotating on a plane parallel to the plane defined by the body (1) of the case.

In the three commented and represented embodiments, the body (1) of the case has a housing (6) formed by a through-hole having a substantially rectangular contour having dimensions complementary to the dimensions of the foldaway handle (2), wherein said foldaway handle (2) can be disposed in a non-unfolded condition.

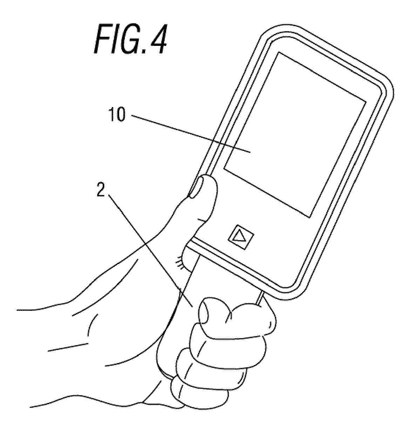

Figure 4 shows an example of application of the case with the foldaway handle (2) held in the user's hand, for example, in a situation ready to take a photograph with the mobile device.

The details, shapes, dimensions and other accessory elements, and the materials used to manufacture the case of the invention, may be conveniently replaced by other technically equivalent ones that do not detract from the essential nature of the invention or from the scope defined by the claims included below.

## Claims

1. A case for mobile phones or similar devices which is formed by a body made of any suitable material having a configuration that fits the mobile phone to be housed, **characterised in that** it comprises a foldaway handle which protrudes from the lower part of the body of the case.

2. The case for mobile phones or similar devices, according to claim 1, **characterised in that** the rear face of the body of the case comprises guiding means through which the handle is susceptible of sliding longitudinally.

3. The case for mobile phones or similar devices, according to claim 2, **characterised in that** the guiding means comprise a pair of longitudinal guides separated from one another, between which the handle slides, said handle having abutting means for limiting the travel path.

4. The case for mobile phones or similar devices, according to claim 1, **characterised in that** one end of the handle comprises a rotary shaft articulated in a hinged manner to the body of the case.

5. The case for mobile phones or similar devices, according to claim 1, **characterised in that** the body of the case includes a shaft wherein the handle is coupled in a swivelling manner, such that said foldaway handle is capable of rotating on a plane parallel to the plane defined by the body of the case.

6. The case for mobile phones or similar devices, according to claim 1, **characterised in that** the body of the case has a housing having dimensions complementary to the dimensions of the foldaway handle, wherein said handle may be disposed in a non-unfolded condition.

7. The case for mobile phones or similar devices, according to claim 6, **characterised in that** the housing consists of a through-hole present in the rear face of the body of the case.

8. The case for mobile phones or similar devices, according to claim 1, **characterised in that** the handle is made of a rigid or semi-rigid material.
